## Europäisches Patentamt
## European Patent Office
## Office européen des brevets

(19)

(11) Numéro de publication: **0 259 420**
**B1**

(12) # FASCICULE DE BREVET EUROPEEN

(45) Date de publication du fascicule du brevet: **23.01.91**

(51) Int. Cl.⁵: **A 61 F 2/34**

(21) Numéro de dépôt: **87901518.8**

(22) Date de dépôt: **10.03.87**

(86) Numéro de dépót international:
**PCT/FR87/00064**

(87) Numéro de publication internationale:
**WO 87/05490 24.09.87 Gazette 87/21**

(54) **CUPULE DE PROTHESE.**

(30) Priorité: **13.03.86 FR 8603778**

(43) Date de publication de la demande:
**16.03.88 Bulletin 88/11**

(45) Mention de la délivrance du brevet:
**23.01.91 Bulletin 91/04**

(84) Etats contractants désignés:
**CH DE FR GB IT LI SE**

(56) Documents cités:
**EP-A-0 091 315**
**EP-A-0 139 356**
**FR-A-2 297 030**
**FR-A-2 493 139**

(73) Titulaire: **BEJUI, Jacques**
**52, rue Lieutenant Colonel Prévost**
**F-69006 Lyon (FR)**
(73) Titulaire: **RHENTER, Jean Luc**
**11, rue de l'Annonciade**
**F-69001 Lyon (FR)**
(73) Titulaire: **Collomb, Jean**
**L'Olagnier**
**F-26800 Portes Les Valence (FR)**

(72) Inventeur: **BEJUI, Jacques**
**52, rue Lieutenant Colonel Prévost**
**F-69006 Lyon (FR)**
Inventeur: **RHENTER, Jean Luc**
**11, rue de l'Annonciade**
**F-69001 Lyon (FR)**
Inventeur: **Collomb, Jean**
**L'Olagnier**
**F-26800 Portes Les Valence (FR)**

(74) Mandataire: **Laurent, Michel et al**
**Cabinet LAURENT et CHARRAS, 20, rue Louis**
**Chirpaz B.P. 32**
**F-69131 Ecully Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Description

L'invention concerne un nouveau type de cupule de prothèse.

Comme on le sait, une prothèse est essentiellement constituée de deux parties, à savoir respectivement d'une part, une tige destinée à être insérée dans l'os, portant à son autre extrémité une tête sur laquelle vient s'articuler la cupule elle-même engagée dans un autre os de l'articulation.

Le plus généralement, à ce jour, la cupule est réalisée en deux parties distinctes, à savoir:

—une cupule dite "de soutien", destinée à être insérée dans un des os de l'articulation à renforcer ou à remplacer;

—l'autre dite "cupule de frottement" destinée à s'insérer dans la cupule de soutien et à recevoir la tête de la tige de prothèse engagée dans un autre os de cette même articulation.

Le plus généralement, la cupule de soutien ou externe est généralement réalisée en métal, notamment en titane ou en alliage de titane, alors que la cupule de frottement interne est réalisée en matière plastique, notamment en polyéthylène (voir par exemple brevets FR—A—2 297 030, 2 493 139 ou européens 0 091 315 et 0 139 356).

L'assemblage entre la cupule externe et la cupule interne est réalisé le plus généralement soit par vissage soit au moyen d'un listel de rétention, soit en faisant appel à des portions mâle et femelle destinées à s'insérer les unes dans les autres lors d'une rotation.

Bref, toutes les dispositions utilisées jusqu'alors font appel à des formes de cupule interne complexes, donc coûteuses, et nécessitent des opérations de mise en place assez délicates exigeant un positionnement précis de l'articulation.

Dans le brevet FR—A—2 493 139 déjà cité, on a décrit une prothèse du type dans laquelle l'intérieur de la rotule de forme sphérique destinée à venir se placer dans le bassin est remplie de polyéthylène. On taille dans ce remplissage amortisseur un alésage pour y insérer un manchon. Lors de la mise en place de la prothèse, le chirurgien engage alors le manchon bloqué dans l'alésage sur l'extrémité conique de la tige. Sous l'effet de l'élévation de la température jusqu'à celle du corps humain, le polyéthylène se dilate pour assurer un bon blocage sur le manchon, qui à son tour est serré par emboitage morse sur l'extrémité de la tige. L'ensemble se trouve ainsi bien immobilisé. Cette solution dans laquelle en fait les frottements s'exercent entre l'os et la rotule métallique présente toutefois les inconvénients:

—d'une part, de n'être adaptée qu'à des prothèses sur bassin sain, puisque la rotule de forme sphérique est placée dans l'os sans y être accrochée,

—d'autre part, qu'à la longue, le remplissage amortisseur en polyéthylène qui ne frotte pas peut se déboiter à l'intérieur de la rotule, ce qui entraîne alors des luxations intraprothétiques.

L'invention pallie ces inconvénients. Elle vise une prothèse du type comportant deux cupules respectivement l'une de soutien, l'autre de frottement, qui soit facile et économique à réaliser en question et surtout facile à mettre en place ou à retirer.

Cette cupule de prothèse constituée en deux parties, à savoir:

. une cupule métallique de soutien destinée à être solidarisée par vissage dans un os de l'articulation à renforcer ou à remplacer, et qui présente en son centre une ouverture cylindrique débouchante;

. une cupule de frottement en matière plastique, destinée à s'insérer dans la cupule de soutien et dont la face interne est demi-sphérique de sorte à pouvoir venir s'emboîter sur la tête sphérique d'une tige de prothèse engagée dans un autre os de cette même articulation; et

. dans lesquelles la forme externe de la cupule de frottement et la forme interne de la cupule de soutien sont complémentaires et s'épousent étroitement; se caractérisé:

—en ce que la forme externe de la cupule de frottement, complémentaire de la forme interne de la cupule de soutien, comprend respectivement:

. à sa base une première portion cylindrique,

. raccordée ensuite à une portion tronconique,

. raccordée elle-même à une portion plane présentant en son centre un têton central cylindrique, épousant la forme de l'ouverture cylindrique débouchante de la cupule de soutien

—en ce que le coefficient de dilatation de la matière plastique constituant la cupule de frottement est, à 20°C au moins vingt fois supérieur à celui du métal constituant la cupule de soutien; et

—en ce que, au jeu près, les cotes externes de la cupule de frottement et les cotes internes de la cupule de soutien coïncident à 0°C.

Avantageusement en pratique, le serrage entre ces deux cupules, respectivement de soutien et de frottement est, à 37°C un serrage positif, compris entre 0,07 mm et 0,005 millimètres, afin d'obtenir le meilleur sertissage; en effet, si le serrage est supérieur, il faudra alors refroidir fortement lors de la mise en place, alors que s'il est inférieur, l'usinage entrainera un coût démesuré sans amélioration notable.

La manière dont l'invention peut être réalisée et les avantages qui en découlent ressortiront mieux de l'exemple de réalisation qui suit, donné à titre indicatif et non limitatif à l'appui des figures annexées.

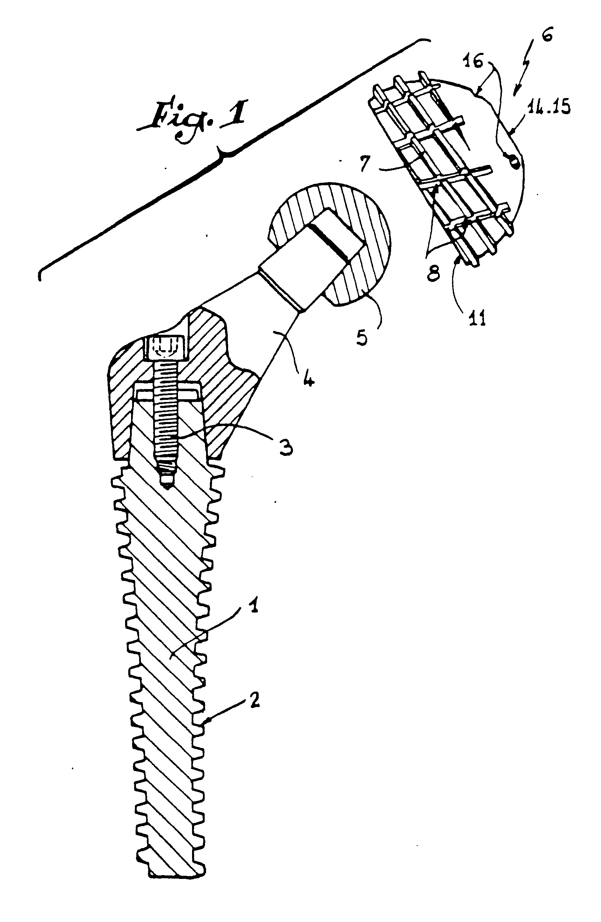

La figure 1 représente une prothèse de hanche ordinaire.

La figure 2 représente en coupe une cupule de frottement conforme à l'invention destinée à être insérée dans une cupule de soutien montrée à la figure 3 également en coupe; cet ensemble étant représenté en figure 4.

La figure 5 montre la cupule de soutien vue de dessous côté base.

Comme on le sait, une prothèse à fixation primaire de hanche comprend essentiellement une tige fémorale (1) filetée (2) pour être insérée dans l'os à renforcer. Cette tige (1) est solidaire par une vis (3) d'une pièce de liaison (4) dont l'extrémité forme une tête (5). Cette tête (5) est destinée à venir coopérer avec la cupule désignée par la référence générale (6) également filetée (7) pour être insérée dans un autre os de la hanche.

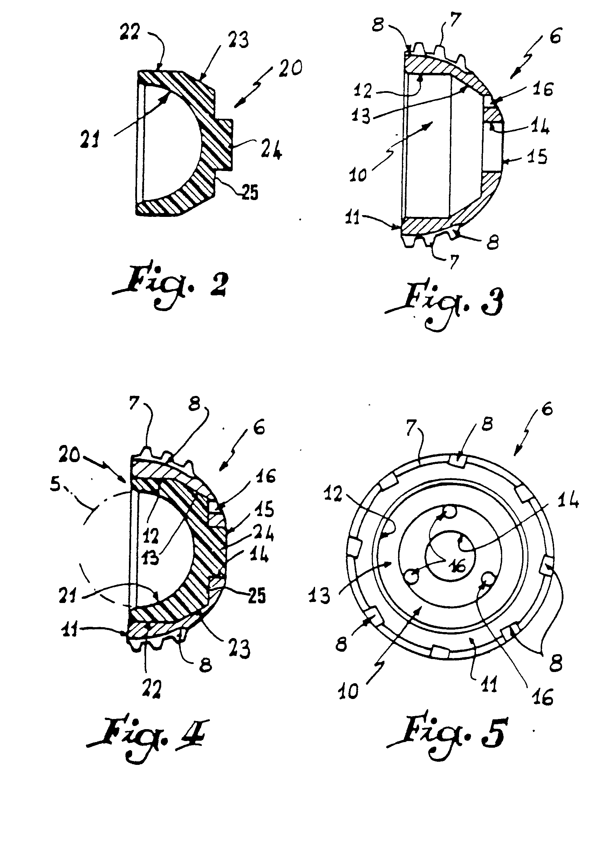

De manière connue, cette cupule (6) montrée aux figures 3 et 4 est filetée (7) et comporte des entailles (8) auto-taraudantes, par exemple au nombre de huit. Cette cupule de soutien externe (6) est réalisée en titane ou en alliage de titane. La face externe de cette cupule est appropriée à l'os qu'elle est destinée renforcer. Selon une caractéristique de l'invention, la face interne (10) de la cupule de soutien présente depuis sa base (11) une première portion cylindrique (12) raccordée à une portion tronconique (13), elle-même raccordée à une portion cylindrique (14) débouchant en (15). Dans une forme de réalisation avantageuse destinée au renforcement du cotyle, le diamètre de la portion cylindrique (12) est de quarante millimètres (alors que le diamètre externe de la cupule (6) est de cinquante-six millimètres), le diamètre de la portion cylindrique (14) et de dix-sept millimètres et la hauteur de l'ensemble (12, 13) est de vingt millimètres, et enfin l'inclinaison de la portion tronconique (13) est de 30°.

La face supérieure de la cupule comporte trois orifices (16) angulairement décalés, destinés à assurer les vissages.

Selon l'invention, la cupule de frottement désignée par la référence générale (20) en polyethylène haute densité, par exemple type RCH 1000, comprend tout d'abord une portion interne (21) demi-cylindrique destinée venir s'emboîter sur la tête (5) de la tige cotyloïdienne (4). Selon un exemple de réalisation avantageux de l'invention, le diamètre externe de la cupule de frottement (20) a un diamètre également de quarante millimètres. La face externe de cette cupule (20) comprend tout d'abord une première portion cylindrique (22) destinée à venir s'emboîter sur (12) raccordée à une portion tronconique (23) destinée à venir s'emboiter en (13) et se termine par un plan horizontal (25) comportant en son centre un têton (24) destiné à venir s'insérer dans l'orifice (15) en plaquant contre les parois (14).

Comme on le sait, le coefficient de dilatation de l'alliage de titane formant la cupule de soutien (6) est de $8,5.10^{-6}$ par degré à 20°C, alors que celui du polyéthylène formant la cupule de frottement (20) est de $210.10^{-6}$ par degré à 20°C.

La cupule externe de soutien (6) peut être conservée à température atmosphérique.

En revanche, on conserve avantageusement la cupule interne (20) de frottement au réfrigérateur, donc dans des conditions stériles.

Lors de l'opération chirurgicale, on met en place la cupule de soutien (6). On sort alors la cupule interne (20) de frottement du réfrigérateur et on l'impacte en (10). Sous l'effet de la température du corps humain, la cupule interne (20) se dilate beaucoup plus rapidement que la cupule externe (6) et vient ainsi se sertir d'elle-même à l'intérieur de (10) en épousant étroitement les parois des portions (12, 13, 14).

Si d'aventure, pour une raison quelconque, le chirurgien doit retirer la cupule, il lui suffit de refroidir la cupule interne (20) pour que celle-ci se retire automatiquement sans aucun problème, par exemple en appliquant une source de froid sur la face (21).

La réalisation selon l'invention se caractérise:

—d'une part, par la simplicité de forme des deux cupules, dont leur facilité d'usinage, ce qui se traduit par un coût financier réduit;

—la facilité de mise en place puisqu'il n'est plus nécessaire de disposer angulairement en rotation la cupule interne et la cupule externe.

La réalisation selon l'invention se distingue de celle décrite dans le brevet FR—A—2 493 139 décrit dans le préambule, par le fait:

—tout d'abord qu'elle est parfaitement adaptée sur un bassin usé et/ou déformé;

—qu'elle est facile à retirer par un simple refroidissement exercé sur la face sphérique (21);

—par l'absence de luxation intraprothétique entre la cupule polyéthylène et la cupule de soutien;

—et enfin, que la cupule de soutien (6) adhère ferment à l'os et qu'une fois en place, il n'y a pas de rotation, de sorte que le blocage est ainsi parfaitement assuré;

—contrairement à l'art antérieur, la cupule polyéthylène assure une fonction de frottement;

—enfin, la mise en place des deux cupules est distincte, ce qui permet de les changer facilement l'une ou l'autre, en particulier la cupule de soutien.

De la sorte, ce type de cupule peut trouver de nombreuses applications dans le domaine des prothèses faisant appel à des cupules de frottement, telles que notamment des prothèses de hanche, de genou, bref pour toute prothèse d'articulation.

**Revendications**

1. Cupule de prothèse, constituée en deux parties, à savoir:

. une cupule métallique de soutien (6), destinée à être solidarisée par vissage (7) dans un os de l'articulation à renforcer ou à remplacer, et qui présente en son centre une ouverture cylindrique (14) débouchante (15);

. une cupule de frottement en matière plastique (20) destinée à s'insérer (10) dans la cupule de soutien (6) et dont la face interne (21) est demi-sphérique de sorte à pouvoir venir s'emboiter sur la tête sphérique (5) d'une tige (1) de prothèse engagée dans un autre os de cette même articulation; et

. dans lesquel les la forme externe (22, 23, 24) de la cupule de frottement (20) et la forme interne (12, 13, 14) de la cupule de soutien (6) sont complémentaires et s'épousent étroitement; caractérisée:

—en ce que la forme externe de la cupule de frottement (20), complémentaire de la forme interne de la cupule de soutien (6), comprend respectivement:

. à sa base une première portion cylindrique (22);

. raccordée ensuite à une portion tronconique (23);

. raccordée elle-même à une portion plane (25) présentant en son centre un têton central (24) cylindrique, épousant la forme de l'ouverture cylindrique (14) débouchante (15) de la cupule de soutien (6);

—en ce que le coefficient de dilatation de la matière plastique constituant la cupule de frottement (20) est, à 20°C, au moins vingt fois supérieur à celui du métal constituant la cupule de soutien (6); et

—en ce que, au jeu près, les cotes externes (22, 23, 24, 25) de la cupule de frottement (20) et les cotes internes (12, 13, 14) de la cupule de soutien (6) coïncident à 0°C.

2. Cupule selon la revendication 1, caractérisée en ce que la cupule de soutien (6) est en titane ou en alliage de titane, alors que la cupule de frottement (20) est en polyéthylène haute densité.

**Patentansprüche**

1. Pfannenprothese, die zwei Teile aufweist, nämlich:

. Eine metallische Stützpfanne (6), die dazu vorgesehen ist, mittels einer Verschraubung (7) zur Verstärkung oder als Ersatz an einem Gelenkknochen befestigt zu werden, und die in ihrer Mitte eine zylindrische Öffnung (14) aufweist, die durchgehend (15) ist;

. eine Reibpfanne aus Plastikmaterial (20), die dazu vorgesehen ist, sich in die Stützpfanne (6) einzufügen (10) und deren innere Fläche (21) derart halbkugelförmig ist, daß sie sich an den kugeligen Kopf (5) eines Prothesenschaftes (1) anpassen kann, der im Eingriff mit einem anderen Knochen desselben Gelenkes ist; und

. bei denen die äußere Gestalt (22, 23, 24) der Reibpfanne (20) und die innere Gestalt (12, 13, 14) der Stützpfanne (6) zueinander komplementär sind und sich eng aneinander anschmiegen; dadurch gekennzeichnet:

—daß die zu der inneren Gestalt der Stützpfanne (6) komplementäre äußere Gestalt der Reibpfanne (20) nacheinander aufweist:

. an ihrer Basis einen ersten zylindrischen Abschnitt (22);

. der unmittelbar mit einem kegelstumpfförmigen Abschnitt (23) verbunden ist;

. welcher selbst mit einem ebenen Abschnitt (25) verbunden ist, der in seiner Mitte einen zylindrischen Mittenansatz (24) aufweist, der zu der Gestalt der zylindrischen Öffnung (14) paßt, die durch die Stützpfanne (6) hindurchgeht (15);

—daß der Dehnungskoeffizient des Plastikmaterials, das die Reibpfanne (20) bildet, bei 20°C wenigstens 20 mal größer ist als derjenige des Metalls, das die Stützpfanne (6) bildet; und

—daß sich die äußeren Kanten (22, 23, 24, 25) der Reibpfanne (20) und die inneren Kanten (12, 13, 14) der Stützpfanne (6) bei 0°C mit geringem Spiel decken.

2. Pfanne nach Anspruch 1, dadurch gekennzeichnet, daß die Stützpfanne (6) aus Titan oder aus einer Titanlegierung besteht, während die Reibpfanne (20) aus Polyäthylen hoher Dichte besteht.

**Claims**

1. A prosthesis cupule constituted in two parts:

—a metallic support cupule (6), designed to be fixed by screwing (7) in a bone of the articulation to be reinforced or replaced, and which is provided in its centre with a cylindrical through (15) opening (14);

—a friction cupule in plastic material (20) designed to be inserted (10) in the support cupule (6), and the internal face (21) of which being semispherical in order to fit over the spherical head (5) of a prosthesis pin (1) inserted in another bone of the same articulation; and

—in which the external shape (22, 23, 24) of the friction cupule (20) is complementary to the internal shape (12, 13, 14) of the support cupule (6) and fit tightly over the latter, characterized:

—in that the external shape of the friction cupule (20), which is complementary to the internal shape of the support cupule (6), respectively comprises:

. at its base, a first cylindrical portion (22);

. joining up with a truncated portion (23);

. which is itself joined to a flat portion (25) provided in its centre with a central cylindrical lug (24), adopting the shape of the cylindrical through (15) opening (14) of the support cupule (6);

—in that the coefficient of expansion of the plastic material which constitutes the friction cupule (20) is, at 20°C, at least twenty times greater than that of the metal constituting the support cupule (6); and

—in that except for some play, the external dimensions (22, 23, 24, 25) of the friction cupule (20) and the internal dimensions (12, 13, 14) of the support cupule (6) coincide at 0°C.

2. A cupule according to claim 1, characterized in that the support cupule (6) is titanium or titanium alloy, and the friction cupule (20) is high density polyethylene.

*Fig. 1*

Fig. 2

Fig. 3

Fig. 4

Fig. 5